# EUROPÄISCHE PATENTANMELDUNG

(11) **EP 3 219 381 A1**
(43) Veröffentlichungstag der Anmeldung: **20.09.2017**
(21) Anmeldenummer: 16160714.8
(22) Anmeldetag: 16.03.2016
(51) Int. Cl.: B01D 69/14, B01D 71/06, B01D 71/76, B01D 71/80, C07K 17/00, C08G 77/452

(54) **PORÖSE DÜNNSCHICHTMEMBRAN, VERFAHREN ZU IHRER HERSTELLUNG SOWIE VERWENDUNGSMÖGLICHKEITEN**

(71) Anmelder: Fraunhofer-Gesellschaft zur Förderung der angewandten Forschung e.V., 80686 München (DE); Rheinisch-Westfälische Technische Hochschule Aachen, 52056 Aachen (DE)
(72) Erfinder: CHARAN, Himanshi, 14469 Potsdam (DE); GLEBE, Ulrich, 14469 Potsdam (DE); BÖKER, Alexander, 13156 Potsdam (DE); TUTUS, Murat, 14469 Potsdam (DE); SCHWANEBERG, Ulrich, 4728 Kelmis-Hergenrath (BE); ZHU, Leilei, Tianjin 300308 (CN); BOCOLA, Marco, 52066 Aachen (DE); MIRZAEIGARAKANI, Tayebeh, 52072 Aachen (DE); KINZEL, Julia, 52068 Aachen (DE); ANAND, Deepak, 52072 Aachen (DE)
(74) Vertreter: Pfenning, Meinig & Partner mbB

(57) **Zusammenfassung**

Gegenstand der Erfindung sind neuartige Membranen, in denen maßgeschneiderte Membrantransport-Proteine (wie z.B. TCDB klassifizierte Proteine) als porenbildende Proteine (z.B. FhuA) oder Peptide, die als Poren in der Membran fungieren. Die Membranen können vorzugsweise sowohl durch das Verlinken von synthetisierten Protein-Polymer-Konjugaten als auch durch direktes Verlinken der porenbildende Proteine und Peptide hergestellt werden. Solche Membranen zeichnen sich durch viele herausstechende Merkmale aus, die bisher existierende Membranen nicht bieten können.

## Beschreibung

Gegenstand der Erfindung sind neuartige Membranen, in denen maßgeschneiderte Membrantransport-Proteine (wie z.B. TCDB klassifizierte Proteine) als porenbildende Proteine (z.B. FhuA) oder Peptide, die als Poren in der Membran fungieren. Die Membranen können vorzugsweise sowohl durch das Verlinken von synthetisierten Protein-Polymer-Konjugaten als auch durch direktes Verlinken der porenbildende Proteine und Peptide hergestellt werden. Solche Membranen zeichnen sich durch viele herausstechende Merkmale aus, die bisher existierende Membranen nicht bieten können.

Eine Klasse von optisch aktiven Verbindungen ("chiral") sind Enantiomere. Von diesen existieren zwei Formen, deren Spiegelbilder identisch sind, die sich aber nicht zur Deckung bringen lassen. Ein sehr anschauliches Beispiel ist die rechte und linke Hand eines Menschen. Für viele Anwendungen wird jedoch eine enantiomerenreine Verbindung benötigt, also nur eine der zwei existierenden Formen. Da diese jedoch häufig in einem Gemisch vorliegen (bei einer 1:1 Mischung spricht man von einem Racemat) und sich chemisch gleich verhalten, wurde eine Reihe von anspruchsvollen Methoden entwickelt, um ein Enantiomerengemisch zu trennen. Enantiomerenreine Verbindungen werden für Arzneistoffe, Nahrungszusatzstoffe, Duftstoffe u.v.m. benötigt.

Durch asymmetrische Synthese lässt sich eine Enantiomerenform direkt synthetisieren. Die Nachteile dabei sind, dass oft keine asymmetrische Synthese existiert und deren Entwicklung mit hohen Kosten verbunden ist sowie geringe Ausbeuten vorliegen.

Zur Trennung eines solchen Gemisches existieren zurzeit vier wesentliche Methoden. Der Review "Membranes and membrane processes for chiral resolution", Chem. Soc. Rev. 2008, 37, 1243 gibt hierzu einen Überblick. Die meisten der gegenwärtig angewendeten Trennmethoden sind entweder kostenintensiv oder ineffizient.

Die bevorzugte Kristallisation eines Enantiomers eignet sich nur für ca. 5-10 % der Racemate.

Außerdem lässt sich eine Form isolieren, indem unterschiedliche Reaktionsraten der beiden Enantiomere mit einer chiralen Einheit ausgenutzt werden. Dies ist jedoch ineffizient und nicht in allen Fällen anwendbar.

Die chromatografische Separation ist zwar breit anwendbar, aber teuer und ineffizient.

Die membranbasierte Trennung hat die Vorteile geringer Kosten, hoher Kapazität, kontinuierlicher Durchführbarkeit und keine speziellen Apparaturen sind notwendig. Sowohl enantioselektive als auch nicht-enantioselektive Membranen existieren bereits zur Trennung von Enantiomerengemischen, diese unterscheiden sich aber grundsätzlich von den hier beschriebenen erfindungsgemäßen Membranen. Theoretische Arbeiten schlagen eine funktionalisierte Graphenschicht als alternative Methode zur Enantiomerentrennung vor (Angew. Chem. Int. Ed. 2014, 53, 9957).

Sämtliche der zuvor genannten Methoden sind jedoch entweder kostenintensiv oder auf eine geringe Substanzmenge beschränkt.

Aus dem Stand der Technik, beispielsweise der US 2011/0046074 A1 sind Membranen bekannt, in die Kanalproteine integriert sind. Die porenbildende Proteine und Peptide sind dabei allerdings nicht kovalent in die Polymermatrix, die die entsprechenden Membranen bildet, integriert, so dass die Stabilität derartiger Membranen gering ist. Zudem sind die entsprechenden Membranen nur für die Wasseraufbereitung vorbeschrieben.

In der Fachliteratur ist das Einfügen von porenbildenden Proteinen und Peptiden in dünne Polymermembranen von Polymersomen und das anschließende Spreiten zu planaren Membranen beschrieben (Small 2012, 8, 1185). Das direkte Verlinken von porenbildenden Proteinen oder Peptiden oder deren Konjugaten ist jedoch bisher noch nicht beschrieben.

Aufgabe der vorliegenden Erfindung ist es daher, stabile und hochfunktionale Polymermembranen mit hoher Porendichte zu ergeben, die zudem vielseitig einsetzbar sein sollen. Die Membranen sollen hierbei u.a. eine simple und kostengünstige Durchführung der Trennung von Enantiomerengemischen mit einem hohen Durchsatz verbinden.

Diese Aufgabe wird hinsichtlich einer porösen Dünnschichtmembran mit den Merkmalen des Patentanspruchs 1, hinsichtlich eines Verfahrens zur Herstellung einer entsprechenden Dünnschichtmembran mit den Merkmalen des Patentanspruchs 12 sowie hinsichtlich entsprechender Verwendungsmöglichkeiten mit den Merkmalen des Patentanspruchs 18 gelöst. Die jeweiligen abhängigen Patentansprüche stellen dabei vorteilhafte Weiterbildungen dar.

Die vorliegende Erfindung betrifft somit eine poröse Dünnschichtmembran aufgebaut aus kovalent vernetzten porenbildenden Proteinen und Peptiden die durchgängige Poren in der Dünnschichtmembran ausbilden..

Der besondere Vorteil von kovalent vernetzten porenbildenden Proteinen oder Peptiden als Poren in Membranen besteht darin, dass die Proteine einheitliche Poren in der Membran formen, die sich darüber hinaus noch funktionalisieren lassen und dadurch vielfältige Anwendungen erlauben. Die Membranen lassen sich energie- und ressourcenschonend auf einfache Art und Weise herstellen. Die Effizienz der Membranen ist durch einen hohen Fluss aufgrund der hohen Anzahl der Kanäle und der sehr dünnen Schichtdicken hoch. Somit lassen sich sowohl kostengünstige als auch effiziente Membranen herstellen. Solche Membranen sind Alternativen zu existierenden Systemen für z. B. die Trennung von Enantiomerengemischen.

Die der vorliegenden Erfindung zugrundeliegenden Prinzipien bedienen sich existierender, großtechnischer Synthesen die z.B. ausschließlich auf die simple Isolierung des gewünschten Enantiomers abgestimmt werden können.

Dabei lassen sich Membranen mit einer wesentlich höheren Dichte an porenbildenden Proteinen oder Peptiden herstellen als über das Einfügen in Polymersom-Membranen. Des Weiteren wird über das direkte Verlinken von Anfang an eine planare Membran aufgebaut, die direkt für Anwendungen eingesetzt werden kann.

Die porenbildenden Proteine oder Peptide dienen als einzigartige einheitliche Poren in der Membran; andere Methoden erlauben zum derzeitigen Stand der Technik nicht die Herstellung von exakt gleich großen Poren im Bereich von wenigen nm Durchmesser. Außerdem ist bei dem beschriebenen Ansatz die Dichte der porenbildenden Proteine oder Peptide in der Membran sehr hoch, was über andere Verfahren ebenfalls nicht gewährleistet werden kann. Die Proteine können sowohl im Innern als auch Äußeren modifiziert werden, so dass verschiedene Funktionalitäten in die Membranen eingebracht werden können. Auf diese Weise können Membranen hergestellt werden, mit denen aufgrund eines ganz spezifisch gestalteten Kanalinneren ein Enantiomerengemisch getrennt werden kann.

Durch die kovalente Vernetzung ist es möglich, dass sehr hohe Porendichten, die durch die jeweiligen porenbildenden Proteine oder Peptide bedingt sind, realisiert werden können. Bevorzugte Porendichten der erfindungsgemäßen Dünnschichtmembranen liegen dabei im Bereich von 1·10⁸ Kanäle/cm² bis 1·10¹³ Kanäle/cm².

Die Porengrößen sind dabei durch das verwendete porenbildende Protein oder Peptid bzw. durch eine ggfs. vorgenommene Funktionalisierung des porenbildenden Proteins oder Peptids bedingt. Typische Porengrößen liegen dabei im Bereich von 0,1 bis 20 nm, bevorzugt von 0,2 bis 10 nm, weiter bevorzugt von 0,25 bis 5 nm und besonders bevorzugt von 0,3 bis 4 nm.

Besonders bevorzugt ist bei der vorliegenden Erfindung, dass die Porengröße aller Poren im Wesentlichen identisch ist, wobei insbesondere die Abweichung der Porengröße kleiner 0,04 nm ist.

Die Dicke der Dünnschichtmembran kann dabei insbesondere zwischen 1 und 100 nm, bevorzugt zwischen 2 und 50 nm, besonders bevorzugt zwischen 3 und 10 nm liegen.

Insbesondere bei extrem dünnen Membranen mit den oben angegebenen Dicken resultiert ein äußerst niedriger Durchflusswiderstand.

Porenbildende Proteine und Peptide, die für die Zwecke der erfindungsgemäßen Dünnschichtmembran verwendet werden können, sind dabei insbesondere ausgewählt aus Transmembranproteinen und/oder Proteinen der TCDB-Klassifikation (http://www.tcdb.org/browse.php) der Kategorien TC #1-9: TC#1) Channels/Pores, TC#2) Electrochemical Potential-driven Transporters, TC#3) Primary Active Transporters, TC#4) Group Translocators, TC#5) Transmembrane Electron Carriers, TC#8) Accessory Factors Involved in Transport, TC#9) Incompletely Characterized Transport Systems. Bevorzugt ist Klasse TC#1 Kanäle/Poren und insbesondere der Klasse 1.B der β-Fassstruktur-Porine wie beispielsweise FhuA der Klasse TC #1.B.14 ein Vetreter the Outer Membrane Receptor (OMR) Familie). Eine Definition der TCDB-Klassifikation findet sich in Saier M.H., Tran C.V., Barabote R.D.: "TCDB: the Transporter Classification Database for membrane transport protein analyses and information", Nucleic Acids Res. 34, Nr. Database issue, Januar 2006, S. D181-D186. Für die Zwecke der vorliegenden Erfindung wird auf die Ausführungen dieses Artikels verwiesen, der durch Bezugnahme in die Offenbarung der vorliegenden Anmeldung mit aufgenommen wird.

Zusätzlich können Kombinationen aus zwei oder mehreren der zuvor genannten porenbildenden Proteine und Peptide verwendet werden.

Die erfindungsgemäßen Dünnschichtmembranen können bevorzugt auf zweierlei Arten hergestellt werden:
Zum Einen durch Vernetzen von Protein/Peptid-Polymer-Konjugaten, die entsprechend vernetzbare Polymerketten tragen.
Eine weitere Möglichkeit betrifft ein direktes Vernetzen der porenbildenden Proteine und Peptide mit einem bi- oder multifunktionalen Linker.

Beide Konzepte werden nachfolgend näher beleuchtet.

Die vorstehend erstgenannte bevorzugte Möglichkeit zur Herstellung der erfindungsgemäßen Dünnschichtmembranen sieht vor, dass die Membran durch das Vernetzen von Protein/Peptid-Polymer-Konjugaten hergestellt wird, wobei die Polymere der Protein/Peptid-Polymer-Konjugate vernetzbare Funktionalitäten aufweisen und insbesondere ausgewählt sind aus der Gruppe bestehend aus Polymeren oder statistischen Copolymeren mit durch Strahlung, radikalische Reaktionen oder Klick-Chemie-Reaktionen vernetzbaren Gruppen bestehen, bevorzugt Poly(co)acrylamiden und Poly(co)acrylaten mit durch Strahlung, radikalische Reaktionen oder Klick-Chemie-Reaktionen vernetzbaren Substituenten, insbesondere Poly(co)(N-isopropylacrylamid)(2-(dimethylmaleimido)-*N*-(ethylacrylamid)), Poly(co)(*N*-isopropylacrylamid)(3,4-dimethyl malein imidobutyl acrylat), Poly(co) (N,N-dimethylaminoethylmethacrylat)(3,4-dimethyl malein imidobutyl methacrylat) oder Poly(co) (vinyl caprolactam) (3,4-dimethyl malein imidobutyl acrylat).

Hierbei ist es insbesondere vorteilhaft, wenn die Polymere der Protein/Peptid-Polymer-Konjugate mittels eines an das porenbildende Protein oder Peptid kovalent gebundenen Initiator, Kettenüberträger oder Katalysator für Ringöffnungs Metathese Polymerisation (ROMP) durch Atom Transfer Radikalische Polymerisation (ATRP), Reversible Additions-Fragmentierungs Kettenübertragungs (RAFT) Polymerisation, Nitroxid-vermittelte Radikalische Polymerisation (NMP), ROMP oder abgewandelte Techniken wie Aktivatoren Generiert durch Elektronenübertragung (AGET) ATRP, Aktivatoren Regeneriert durch Elektronenübertragung (ARGET) ATRP, Einzelelektronenübertragungs Lebende Radikalische Polymerisation (SET-LRP) oder Zusätzliche Aktivatoren und Reduzierungs Agenzien Atom Transfer Radikalische Polymerisation (SARA ATRP) an das porenbildende Protein oder Peptid kovalent angebunden sind bzw. werden. Beispielsweise kann an eine Aminogruppe des porenbildenden Proteins oder Peptids Succinimidyl-3-(2-brom-2-methylpropionamido)-propionat als Initiator gebunden werden.

Alternativ können die erfindungsgemäßen Dünnschichtmembranen bevorzugt ebenso durch Vernetzen von kanonischen und nicht-kanonischen Aminosäurenresten oder glykosylierten Positionen der porenbildenden Proteine oder Peptide mittels mindestens eines bi- oder multifunktionellen Vernetzers hergestellt werden, wobei der Vernetzer bevorzugt ausgewählt ist aus der Gruppe bestehend aus Dialdehyden, Dicarbonsäuren, *N-*Hydroxysuccinimid-aktivierten Dicarbonsäuren, Disäurehalogeniden, Diaminen und Diiso(thio)cyanaten wie z.B. 1,3-Propiondial, 1,4-Butandial oder 1,5-Pentandial.

Ebenso ist es allerdings möglich, dass beide der zuvor genannten reaktiven Prinzipien zur Herstellung der erfindungsgemäßen Dünnschichtmembran angewandt werden, so dass beispielsweise auch direkt miteinander vernetzte porenbildende Proteine oder Peptide neben über vernetzte Polymerketten verknüpfte porenbildende Proteine oder Peptide in der Dünnschichtmembran vorliegen können.

Ebenso ist es möglich, dass die porenbildenden Proteine und Peptide an der inneren Porenoberfläche funktionalisiert sind, insbesondere mit Gruppen, die die Porengröße variieren oder mit Ladungen versehen.

Bevorzugt ist weiterhin dass die Dünnschichtmembran auf einer porösen Trägerstruktur, insbesondere ausgewählt aus der Gruppe bestehend aus Cellulosenitrat-Membranen, Celluloseacetat-Membranen, Nitrocellulose-Membranen, Mixed Cellulose Ester Membranen, PTFE (Polytetrafluorethylen)-Membranen, Polyethersulfon (PES)-Membranen, Regenerierte Cellulose-Membranen, Polycarbonat-Membranen, Polyamid-Membranen und Polyacrylnitril-Membranen aufgebracht ist.

Die Erfindung betrifft zudem ein Verfahren zur Herstellung einer porösen Dünnschichtmembranen nach einem der vorhergehenden Ansprüche, bei dem porenbildende Proteine und Peptide kovalent miteinander vernetzt werden.

Gemäß einer ersten bevorzugten Ausführungsform des erfindungsgemäßen Verfahrens ist vorgesehen,
a) dass mindestens ein Initiator an jedes porenbildende Protein oder Peptid über mindestens einen Aminosäurerest kovalent gebunden wird, beispielsweise Succinimidyl-3-(2-brom-2-methylpropionamido)-propionat an eine Aminogruppe mindestens einer Aminosäure jedes Porenbildende Proteins oder Peptids kovalent gebunden wird,
b) das Initiator-, Kettenüberträger- oder Katalysator-funktionalisierte porenbildende Protein oder Peptid mit Monomeren zur Reaktion gebracht wird, wobei Protein/Peptid-Polymer-Konjugate gebildet werden, bei denen Polymere oder statistische Copolymere mit durch Strahlung, radikalische Reaktionen oder Klick-Chemie-Reaktionen vernetzbaren Gruppen, bevorzugt Poly(co)acrylamide und Poly(co)acrylate mit durch Strahlung, radikalische Reaktionen oder Klick-Chemie-Reaktionen vernetzbaren Substituenten, insbesondere Poly(co)(*N*-isopropylacrylamid)(2-(dimethylmaleimido)-*N-*(ethylacrylamid)), Poly(co)(*N*-isopropylacrylamid)(3,4-dimethyl malein imidobutyl acrylat), Poly(co) (N,N-dimethylaminoethylmethacrylat)(3,4-dimethyl malein imidobutyl methacrylat) oder Poly(co) (vinyl caprolactam) (3,4-dimethyl malein imidobutyl acrylat) mittels eines Initiators, Kettenüberträgers oder Katalysators für ROMP durch ATRP, RAFT Polymerisation, NMP, ROMP oder abgewandelte Techniken wie AGET ATRP, ARGET ATRP, SET-LRP oder SARA ATRP kovalent an das porenbildende Protein oder Peptid gebunden werden, und anschließend
c) eine Vernetzung der Protein/Peptid-Polymer-Konjugate, insbesondere durch Strahlung (z.B. UV-Strahlung), radikalische Reaktionen oder Klick-Chemie-Reaktionen durchgeführt wird.

Alternativ oder zusätzlich zu der zuvor genannten Vorgehensweise ist es ebenso möglich, dass die porenbildenden Proteine und Peptide über kanonische und nicht-kanonische Aminosäurenreste oder glykosylierte Positionen mit mindestens einem bi- oder multifunktionellen Vernetzer, bevorzugt einem Vernetzer ausgewählt aus der Gruppe bestehend aus Dialdehyden, Dicarbonsäuren, *N*-Hydroxysuccinimid-aktivierten Dicarbonsäuren, Disäurehalogeniden, Diaminen und Diiso(thio)cyanaten wie z.B. 1,3-Propiondial, 1,4-Butandial oder 1,5-Pentandial kovalent vernetzt werden.

Die porenbildenden Proteine und Peptide können bei der erfindungsgemäßen Verfahrensweise beispielsweise auch an der inneren Porenoberfläche funktionalisiert werden, insbesondere mit Gruppen, die die Porengröße variieren oder mit Ladungen versehen.

Dabei kann die Funktionalisierung der inneren Porenoberfläche zu jedem beliebigen Zeitpunkt der Verfahrensführung vorgenommen werden, beispielsweise auch vor oder aber erst nach der Vernetzung.

Eine weitere bevorzugte Ausführungsform des erfindungsgemäßen Verfahrens sieht vor, dass die Protein/Peptid-Polymer-Konjugate bzw. die porenbildenden Proteine und Peptide vor der Vernetzung an einer Grenzfläche assembliert werden, wobei die Grenzfläche bevorzugt eine flüssig-flüssig Grenzfläche oder die Wasser/Luft-Grenzfläche eines Tropfens auf der Oberfläche einer porösen Trägerstruktur, insbesondere ausgewählt aus der Gruppe bestehend aus Cellulosenitrat-Membranen, Celluloseacetat-Membranen, Nitrocellulose-Membranen, Mixed Cellulose Ester Membranen, PTFE (Polytetrafluorethylen)-Membranen, Polyethersulfon (PES)-Membranen, Regenerierte Cellulose-Membranen, Polycarbonat-Membranen, Polyamid-Membranen und Polyacrylnitril-Membranen darstellt.

Insbesondere erfolgt die Vernetzung sowie ggf. das Anbinden des Initiators, Kettenüberträgers oder ROMP Katalysators und/oder die Herstellung des Protein/Peptid-Polymer-Konjugates in wässriger Lösung. Die wässrige Lösung kann dabei neben Wasser und den Reaktanden auch gewisse Mengen organischer Lösungsmittel, Detergenzien oder oberflächenaktive Stoffe aufweisen.

Die Polymermatrix der Membranen kann durch Verwendung hydrophober Monomere für die Polymerisation, durch Einstellen bestimmter Temperaturen bei Verwendung von thermoresponsiven Polymeren oder durch Verändern des pH-Werts bei Verwendung von pH-responsiven Polymeren so beeinflusst werden, dass die Polymermatrix hydrophob ist oder wird und der Wasserfluss und der Fluss hydrophiler Moleküle bevorzugt durch die Protein-/Peptidkanäle und nicht die Polymermatrix geht.

Zudem betrifft die vorliegende Erfindung Verwendungsmöglichkeiten der erfindungsgemäßen Dünnschichtmembran. Insbesondere eignet sich die Dünnschichtmembran zur Separation von Molekülen, insbesondere nach Ladung, Größe, chemischer Zusammensetzung, intermolekularen Wechselwirkungen und Chiralität, bevorzugt zur Trennung von Enantiomeren oder zur Wasseraufbereitung, insbesondere zur Wasserentsalzung.

Die vorliegende Erfindung wird anhand der nachfolgenden Ausführungen und Erläuterungen näher dargestellt, ohne die Erfindung auf die dargestellten speziellen Ausführungen zu beschränken.

Hierbei zeigen:
Figur 1 eine schematische Aufsicht auf eine planare Membran gemäß der vorliegenden Erfindung
Figur 2 eine beispielhafte Synthesemöglichkeit zur Herstellung einer erfindungsgemäßen Dünnschichtmembran
Figur 3 die Prinzipien zur Herstellung einer erfindungsgemäßen Dünnschichtmembran auf einer porösen Trägeroberfläche
Figur 4 einen Einblick in einen modifizierten Kanal eines porenbildenden Proteins oder Peptids einer erfindungsgemäßen Membran für die Anwendungsmöglichkeit zur Trennung von Enantiomerengemischen.

Bislang existiert kein Verfahren, um Membranen mit großer Dichte an von porenbildenden Proteinen oder Peptiden, insbesondere Transmembranproteinen, die durchgängige Poren in der Dünnschichtmembran ausbilden mit exakt einheitlicher Größe im Bereich weniger nm herzustellen, das auf einem anderen Weg als dem Ausnutzen der Einförmigkeit von porenbildenden Proteinen oder Peptiden beruht.

Zudem existieren keine Membranen, in denen die porenbildenden Proteine und Peptide kovalent gebunden sind, so dass durchgängige Poren in der Dünnschichtmembran ausgebildet werden.

Dieser Missstand wird durch die vorliegende Erfindung beseitigt.

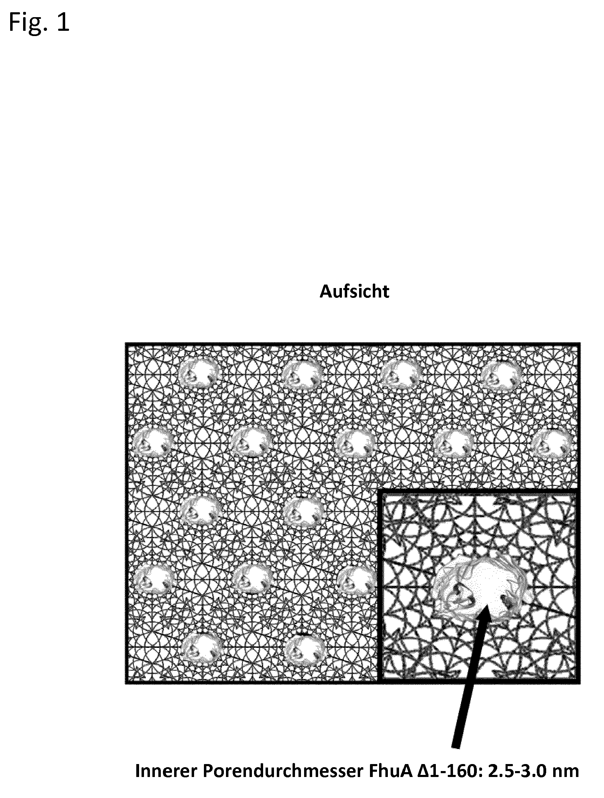

Figur 1 zeigt eine Aufsicht auf eine planare Dünnschichtmembran gemäß der vorliegenden Erfindung, mit hoher Dichte an verlinkten porenbildenden Proteinen oder Peptiden. Die porenbildenden Proteine und Peptide weisen dabei typischerweise einen Poreninnendurchmesser von 1 bis 3 nm auf und sind über ein Netzwerk von Polymeren miteinander kovalent verbunden. Alternativ hierzu (nicht dargestellt) können die porenbildenden Proteine und Peptide auch direkt über bi- oder multifunktionelle, monomolekulare Linker miteinander verknüpft sein.

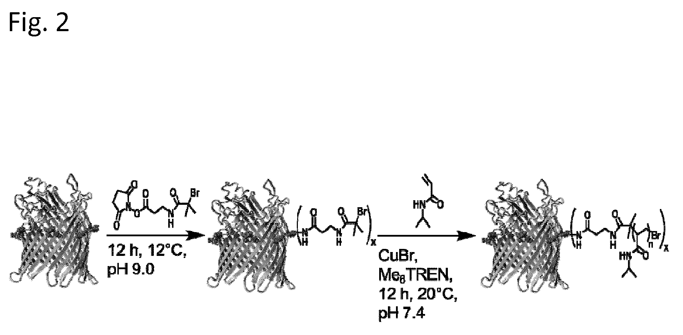

Membranen, in denen porenbildende Proteine und Peptide wie FhuA mit geöffnetem Kanal von ca. 1-3 nm Durchmesser mit hoher Dichte sitzen, lassen sich auf zwei verschiedene Weisen herstellen: durch Vernetzen der Polymerketten von Protein/Peptid-Polymer-Konjugaten (Fig. 2) sowie durch direktes Vernetzen der porenbildenden Proteine und Peptide mit einem bi- oder multifunktionalen Linker.

Die Darstellung von Protein/Peptid-Polymer Konjugaten ist vielzählig mit globulären und löslichen Proteinen sowie Viren beschrieben (einen Überblick geben Polym. Chem. 2015, 6, 5143 und Chem. Commun. 2011, 47, 2212). Die Konjugatsynthese mit porenbildenden Proteinen oder Peptiden durch den *grafting-from* Ansatz ist jedoch nicht bekannt. Von den verschiedenen Strategien zur Synthese von Protein/Peptid-Polymer Konjugaten hat das verwendete *grafting-from* den Vorteil, dass eine vergleichsweise hohe Anzahl an Polymerketten von der Protein-/Peptidoberfläche gewachsen werden kann.

Figur 2 zeigt die beispielhafte Synthese von Protein/Peptid-Polymer Konjugaten durch Anbinden einer Initiatoreinheit an Aminosäurereste (links) und anschließende Polymersynthese von der Protein-/Peptidoberfläche. Im Beispiel ist die Copolymerisation von *N*-Isopropylacrylamid (NIPAAm) mit ca. 5 % 2-(Dimethylmaleimido)-N-ethylacrylamid (DMIAAm) gezeigt. Die Seitenketten von DMIAAm sind in einer [2+2]-Cycloaddition unter UV-Licht vernetzbar. Durch die mittels UV-Licht erfolgende Vernetzung wird dabei die finale Membran erzeugt.

Für die in Fig. 2 gezeigte Synthese wird zunächst ein Initiator für die Polymerisation an einen Aminosäurerest gebunden, im Beispiel an die Lysinreste von FhuA. Die anschließende Polymerisation ist von NIPAAm gezeigt, kann aber auch mit anderen Monomeren erfolgen. Um das Vernetzen der Polymerketten zu ermöglichen, wird ein entsprechendes Comonomer wie DMIAAm in etwa 5 % hinzugegeben. Die Maleimid-Einheiten können durch Bestrahlung mit UV-Licht in einer [2+2]-Cycloaddition Polymerketten verlinken.

Porenbildende Proteine und Peptide haben durch ihre hydrophilen und hydrophoben Bereiche eine intrinsische Grenzflächenaktivität. Im Vergleich zu unmodifizierten Proteinen ist die Grenzflächenaktivität von Protein/Peptid-Polymer Konjugaten in der Regel nochmals deutlich höher. Aus stark verdünnter Lösung selbstassemblieren die Konjugate an der Luft-Wasser-Grenzfläche und können durch Verlinken der Polymerketten untereinander zu einer stabilen, dünnen Membran verbunden werden. Nach Verdampfen der WasserPhase liegt diese Membran planar auf dem verwendeten Support auf.

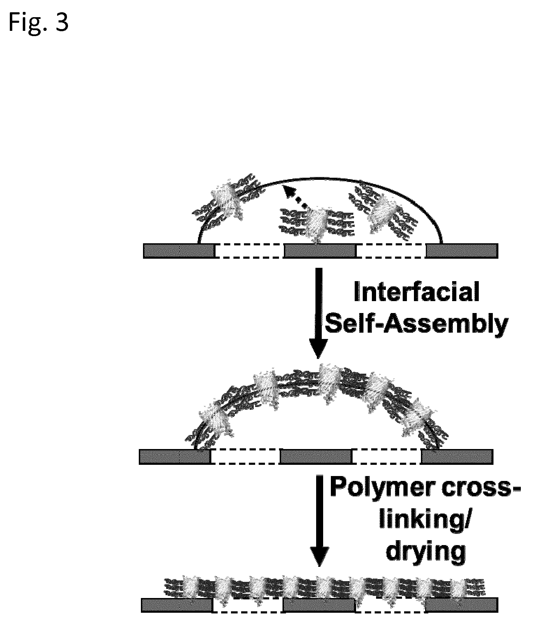

In diesem Zusammenhang zeigt beispielsweise Figur 3 das Prinzip der Selbstassemblierung von Membranprotein-Polymer Konjugaten an der Wasser-Luft-Grenzfläche und Verlinken der Polymerketten. Nach Verdampfen des Wassers sitzt die Membran auf einem porösen Träger.

Neben dem Verlinken von kovalent angebundenen Polymerketten können porenbildende Proteine und Peptide auch direkt durch Vernetzer verlinkt werden. Die Vernetzer müssen über mindestens zwei von einem kurzen Abstandshalter getrennten Funktionalitäten verfügen, die mit Aminosäureresten reagieren. Ein Beispiel ist Glutaraldehyd, welches mit den Aminogruppen der Aminosäure Lysin reagiert. Auf diese Weise ist der Abstand der porenbildende Proteine oder Peptide in der Membran nochmals geringer und die größtmögliche Dichte der Proteinporen lässt sich erreichen.

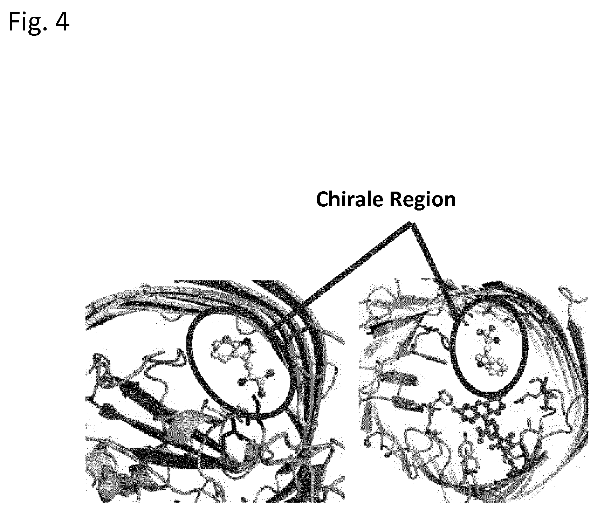

Eine mögliche Anwendung solcher Membranen ist die Nutzung zur Trennung von Enantiomerengemischen. Die Komponenten sind kleiner als der Kanaldurchmesser, um einen Durchfluss der Enantiomere zu gewährleisten, wobei es bevorzugt nur einem Enantiomer erlaubt ist, den Kanal zu passieren. Dies wird durch chemische und/oder genetische Modifikationen im Kanalinneren erreicht, welche unterschiedlich mit den verschiedenen Enantiomeren interagieren. Mögliche Stoffklassen sind enantiomere Aminosäuren aber auch Amine, Epoxide und Terpene. Fig. 4 zeigt beispielhaft Tryptophan im Kanal für eine chirale Trennung von Aminosäuren.

Figur 4 zeigt dabei verschiedene Strategien zum FhuA-Engineering um A) sterisch nicht anspruchsvolle (FhuA-Wildtyp) und B) sterisch anspruchsvolle (FhuA Δ1-159 mit Fluorescein-Markierung) Enantiomerengemische zu trennen (Querschnittsansicht von FhuA).

Technische Membranen mit kovalent gebundenen porenbildenden Proteinen oder Peptiden als Poren sind eine neue Klasse von Membranen. Das Hauptanwendungsgebiet sind Separationen aller Art. Durch die einheitliche Größe der Protein-/Peptidkanäle ist zunächst eine Separation aufgrund eines Größenausschlusses in bisher nicht erreichter Präzision möglich. Partikel, deren Größe unter den 2-3 nm Durchmesser der Poren liegen, können die Membran passieren, während größere Partikel zurückgehalten werden. Zudem ermöglicht der niedrige Durchflusswiderstand bisher unerreichte Flüsse über die Membran bei niedrigem Energiebedarf. Durch das Einbringen von Funktionalitäten in das Kanalinnere können Separationen durchgeführt werden, die über einen reinen Größenausschluss hinausgehen. Hier liegt ein wesentliches Anwendungsgebiet in der Trennung eines Enantiomerengemisches. Die Membranen erlauben also einen neuen Ansatz zur Herstellung enantiomerenreiner Verbindungen, welcher gegenüber den existierenden Verfahren einen deutlichen Kosten- und Effizienz-Vorteil bietet.

## Patentansprüche

1. Poröse Dünnschichtmembran aufgebaut aus kovalent vernetzten porenbildenden Proteinen oder Peptiden, die durchgängige Poren in der Dünnschichtmembran ausbilden.

2. Dünnschichtmembran nach Anspruch 1, **dadurch gekennzeichnet, dass** die Dünnschichtmembran eine Porendichte im Bereich von 1·10⁸ Kanäle/cm² bis 1·10¹³ Kanäle/cm² aufweist.

3. Dünnschichtmembran nach einem der vorhergehenden Ansprüche, **dadurch gekennzeichnet, dass** die Porengröße im Bereich von 0,1 bis 20 nm, bevorzugt von 0,2 bis 10 nm, weiter bevorzugt von 0,25 bis 5 nm und besonders bevorzugt von 0,3 bis 4 nm liegt.

4. Dünnschichtmembran nach einem der vorhergehenden Ansprüche, **dadurch gekennzeichnet, dass** die Porengröße aller Poren im Wesentlichen identisch ist, wobei insbesondere die Abweichung der Porengröße kleiner 0,04 nm ist.

5. Dünnschichtmembran nach einem der vorhergehenden Ansprüche, **dadurch gekennzeichnet, dass** die Dicke der Dünnschichtmembran zwischen 1 und 100 nm, bevorzugt zwischen 2 und 50 nm, besonders bevorzugt zwischen 3 und 10 nm beträgt.

6. Dünnschichtmembran nach einem der vorhergehenden Ansprüche, **dadurch gekennzeichnet, dass** die porenbildenden Proteine oder Peptide ausgewählt sind aus der Gruppe bestehend aus Transmembranproteinen, Proteinen oder Peptiden der TCDB-Klassifikation-Kategorien TC#1-9, bevorzugt der Klasse TC#1 Kanäle/Poren und insbesondere der Klasse TC#1.B der β-Fassstruktur-Porine wie beispielsweise FhuA der Klasse TC #1.B.14 ein Vertreter der Outer Membrane Receptor (OMR) Familie) sowie Kombinationen aus zwei oder mehreren der zuvor genannten porenbildenden Proteinen oder Peptiden.

7. Dünnschichtmembran nach einem der vorhergehenden Ansprüche, **dadurch gekennzeichnet, dass** die Membran durch das Vernetzen von Protein/Peptid-Polymer-Konjugaten hergestellt wird, wobei die Polymere der Protein/Peptid-Polymer-Konjugate vernetzbare Funktionalitäten aufweisen und insbesondere ausgewählt sind aus der Gruppe bestehend aus Polymeren oder statistischen Copolymeren mit durch Strahlung, radikalische Reaktionen oder Klick-Chemie-Reaktionen vernetzbaren Gruppen bestehen, bevorzugt Poly(co)acrylamiden und Poly(co)acrylaten mit durch Strahlung, radikalische Reaktionen oder Klick-Chemie-Reaktionen vernetzbaren Substituenten, insbesondere Poly(co)(*N*-isopropylacrylamid)(2-(dimethylmaleimido)-*N*-(ethyl-acrylamid)), Poly(co)(*N*-isopropylacrylamid)(3,4-dimethyl malein imidobutyl acrylat), Poly(co) (*N,N*-dimethylaminoethylmethacrylat)-(3,4-dimethyl malein imidobutyl methacrylat) oder Poly(co) (vinyl caprolactam) (3,4-dimethyl malein imidobutyl acrylat).

8. Dünnschichtmembran nach einem der beiden vorhergehenden Ansprüche, **dadurch gekennzeichnet, dass** die Polymere der Protein/Peptid-Polymer-Konjugate mittels eines an das porenbildende Protein oder Peptid kovalent gebundenen Initiator, Kettenüberträger oder Katalysator für Ringöffnungs Metathese Polymerisation (ROMP) durch Atom Transfer Radikalische Polymerisation (ATRP), Reversible Additions-Fragmentierungs Kettenübertragungs (RAFT) Polymerisation, Nitroxid-vermittelte Radikalische Polymerisation (NMP), ROMP oder abgewandelte Techniken wie Aktivatoren Generiert durch Elektronenübertragung (AGET) ATRP, Aktivatoren Regeneriert durch Elektronenübertragung (ARGET) ATRP, Einzelelektronenübertragungs Lebende Radikalische Polymerisation (SET-LRP) oder Zusätzliche Aktivatoren und Reduzierungs Agenzien Atom Transfer Radikalische Polymerisation (SARA ATRP) an das porenbildende Protein oder Peptid kovalent angebunden sind bzw. werden, insbesondere mittels Succinimidyl-3-(2-brom-2-methylpropionamido)-propionat.

9. Dünnschichtmembran nach einem der vorhergehenden Ansprüche, **dadurch gekennzeichnet, dass** die Membran durch Vernetzen von Aminosäurenresten oder Glykosylierungsgruppen der porenbildenden Proteine oder Peptide mittels mindestens eines bi- oder multifunktionellen Vernetzers hergestellt wird, wobei der Vernetzer bevorzugt ausgewählt ist aus der Gruppe bestehend aus Dialdehyden, Dicarbonsäuren, *N*-Hydroxysuccinimid-aktivierten Dicarbonsäuren, Disäurehalogeniden, Diaminen und Diiso(thio)cyanaten wie z.B. 1,3-Propiondial, 1,4-Butandial oder 1,5-Pentandial.

10. Dünnschichtmembran nach einem der vorhergehenden Ansprüche, **dadurch gekennzeichnet, dass** die porenbildenden Proteine oder Peptide an der inneren Porenoberfläche funktionalisiert sind, insbesondere mit Gruppen, die die Porengröße variieren oder mit Ladungen versehen.

11. Dünnschichtmembran nach einem der vorhergehenden Ansprüche, **dadurch gekennzeichnet, dass** die Dünnschichtmembran auf einer porösen Trägerstruktur, insbesondere ausgewählt aus der Gruppe bestehend aus Cellulosenitrat-Membranen, Celluloseacetat-Membranen, Nitrocellulose-Membranen, Mixed Cellulose Ester Membranen, PTFE (Polytetrafluorethylen)-Membranen, Polyethersulfon (PES)-Membranen, Regenerierte Cellulose-Membranen, Polycarbonat-Membranen, Polyamid-Membranen und Polyacrylnitril-Membranen aufgebracht ist.

12. Verfahren zur Herstellung einer porösen Dünnschichtmembran nach einem der vorhergehenden Ansprüche, bei dem porenbildende Proteine oder Peptide kovalent miteinander vernetzt werden.

13. Verfahren nach vorhergehendem Anspruch, bei dem
a) mindestens ein Initiator, Kettenüberträger oder Katalysator für ROMP an jedes porenbildende Protein oder Peptid über mindestens einen Aminosäurerest kovalent gebunden wird, beispielsweise Succinimidyl-3-(2-brom-2-methylpropionamido)-propionat an eine Aminogruppe mindestens einer Aminosäure jedes porenbildenden Proteins oder Peptids kovalent gebunden wird,
b) das Initiator-, Kettenüberträger- oder Katalysator- funktionalisierte porenbildende Protein oder Peptid mit Monomeren zur Reaktion gebracht wird, wobei Protein/Peptid-Polymer-Konjugate gebildet werden, bei denen Polymere oder statistische Copolymere mit durch Strahlung, radikalische Reaktionen oder Klick-Chemie-Reaktionen vernetzbaren Gruppen, bevorzugt Poly(co)acrylamide und Poly(co)acrylate mit durch Strahlung, radikalische Reaktionen oder Klick-Chemie-Reaktionen vernetzbaren Substituenten, insbesondere Poly(co)(*N*-isopropylacrylamid)(2-(dimethylmaleimido)-*N-*(ethylacrylamid)), Poly(co)(*N*-isopropylacrylamid)(3,4-dimethyl malein imidobutyl acrylat), Poly(co) (N,N-dimethylaminoethyl-methacrylat)(3,4-dimethyl malein imidobutyl methacrylat) oder Poly(co) (vinyl caprolactam) (3,4-dimethyl malein imidobutyl acrylat) mittels eines Initiators, Kettenüberträgers oder Katalysators für ROMP durch ATRP, RAFT Polymerisation, NMP, ROMP oder abgewandelte Techniken wie AGET ATRP, ARGET ATRP, SET-LRP oder SARA ATRP kovalent an das porenbildende Protein oder Peptid gebunden werden, und anschließend
c) eine Vernetzung der Protein/Peptid-Polymer-Konjugate, insbesondere durch Strahlung, radikalische Reaktionen oder Klick-Chemie-Reaktionen durchgeführt wird.

14. Verfahren nach einem der Ansprüche 12 bis 13, bei dem die porenbildenden Proteine und Peptide über die Aminosäurereste mit mindestens einem bi- oder multifunktionellen Vernetzer, bevorzugt einem Vernetzer ausgewählt aus der Gruppe bestehend aus Dialdehyden, Dicarbonsäuren, Disäurehalogeniden und Diaminen wie z.B. 1,3-Propiondial, 1,4-Butandial oder 1,5-Pentandial kovalent vernetzt werden.

15. Verfahren nach einem der Ansprüche 12 bis 14, **dadurch gekennzeichnet, dass** die porenbildenden Proteine oder Peptide an der inneren Porenoberfläche funktionalisiert werden, insbesondere mit Gruppen, die die Porengröße variieren oder mit Ladungen versehen.

16. Verfahren nach einem der Ansprüche 12 bis 15, **dadurch gekennzeichnet, dass** die Protein/Peptid-Polymer-Konjugate bzw. die porenbildenden Proteine oder Peptide vor der Vernetzung an einer Grenzfläche assembliert werden, wobei die Grenzfläche bevorzugt eine flüssig-flüssig Grenzfläche oder die Wasser/Luft-Grenzfläche eines Tropfens auf der Oberfläche einer porösen Trägerstruktur, insbesondere ausgewählt aus der Gruppe bestehend aus Cellulosenitrat-Membranen, Celluloseacetat-Membranen, Nitrocellulose-Membranen, Mixed Cellulose Ester Membranen, PTFE (Polytetrafluorethylen)-Membranen, Polyethersulfon (PES)-Membranen, Regenerierte Cellulose-Membranen, Polycarbonat-Membranen, Polyamid-Membranen und Polyacrylnitril-Membranen darstellt.

17. Verfahren nach einem der Ansprüche 12 bis 16, **dadurch gekennzeichnet, dass** die Vernetzung sowie ggf. das Anbinden des Initiators und/oder die Herstellung des Protein/Peptid-Polymer-Konjugates in wässriger Lösung erfolgt.

18. Verwendung der porösen Dünnschichtmembran nach einem der Ansprüche 1 bis 11 zur Separation von Molekülen, insbesondere nach Ladung, Größe, chemischer Zusammensetzung, intermolekularen Wechselwirkungen und Chiralität, bevorzugt zur Trennung von Enantiomeren oder zur Wasseraufbereitung, insbesondere zur Wasserentsalzung.
